(19) Europäisches Patentamt
European Patent Office
Office européen des brevets

(11) Publication number: 0 011 608
A1

(12) EUROPEAN PATENT APPLICATION

(21) Application number: 79850076.5

(22) Date of filing: 21.08.79

(51) Int. Cl.³: C 07 C 87/29
A 61 K 31/135

(30) Priority: 30.08.78 SE 7809127

(43) Date of publication of application: 28.05.80 Bulletin 80/11

(84) Designated Contracting States: BE CH DE FR GB IT LU NL

(71) Applicant: Astra Läkemedel Aktiebolag
Strängnäsvägen 44
S-151 85 Södertälje(SE)

(72) Inventor: Claesson, Alf
Täljstensvägen 16B
S-752 40 Uppsala(SE)

(72) Inventor: Ross, Svante Bertil
Hedvägen 8
S-151 52 Södertälje(SE)

(72) Inventor: Sahlberg, Christer
Lohäradsvägen 9
S-194 00 Upplands-Väsby(SE)

(74) Representative: Wurm, Bengt R. et al,
S-151 85 Södertälje(SE)

(54) **Novel allenic amine and acid addition salts thereof, a process for their preparation, pharmaceutical preparations comprising them and the use of these compounds in the preparation of pharmaceutical compositions.**

(57) Novel allenic amine of the formula

(I) 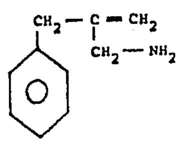

and the pharmaceutically acceptable acid-addition salts thereof exhibit monoamine oxidase enzyme inhibiting and antidepressive activity. The novel compound (I) is prepared on the following way:

$CH_3$-O-$CH_2$-C≡CH + $CH_2O$ + $HNBu_2$ $\xrightarrow[Dioxane]{Cu_2Cl}$ CH3-O-$CH_2$-C≡C-$CH_2$-$NBu_2$

$\xrightarrow[Et_2O]{BrCN}$ $CH_3$O-$CH_2$-C≡C-$CH_2$Br $\xrightarrow{NH_3}$ $CH_3$-O-$CH_2$-C≡C-$CH_2$-$NH_2$

$\xrightarrow[Et_2O,\ Cu_2Br_2]{C_6H_5CH_2MgCl}$ (I)

EP 0 011 608 A1

0011608

Astra Läkemedel Aktiebolag
Södertälje/Sweden

Inventors: A Claesson, S Ross and C Sahlberg

LA 586-1
79 08 20
IM/RD

Allenic Amines

TITLE MODIFIED
see front page

Description

Technical Field

This invention relates to novel allenic amines and to methods for their preparation as well as to pharmaceutical compositions containing the allenic amines and to the use of the allenic amines for the preparation of pharmaceutical compositions.

Background Art

Depressive disorders have with more or less success been treated with various compounds. Many types of chemical substances have been used, among these amphetamine with the structure

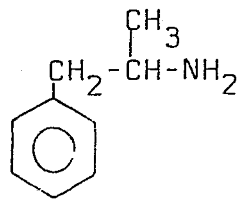

However, the euphoretic effect and the risk for dependence have to a great extent restricted the use of amphetamine in the therapy.

The medical use of amphetamine is nowadays mainly restricted to the treatment of narcolepsy and asthenic states in aged people.

A further type of chemical substances that have been suggested for use as an antidepressive agent includes monoamine oxidase enzyme inhibiting chemical compounds, for example compounds of the formula

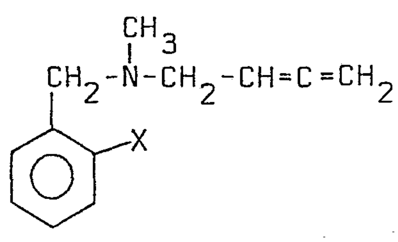

wherein X is hydrogen or chloro. These compounds are disclosed in Journal of Pharmaceutical Sciences, Vol. 57, No. 3, March 1968, p 430-433, and in United States Patent No. 3,397,236.

Disclosure of invention

We have found that novel allenic amines of the formula

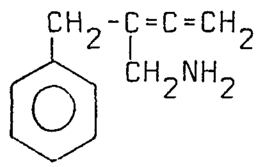

and pharmaceutically acceptable acid-addition salts thereof, exhibit a higher monoamine oxidase enzyme (MAO) inhibiting activity than the above-mentioned known allenic amines. The pharmacological profile of the compounds of the invention suggests a potential value of the compounds as antidepressants, and in the treatment of Parkinson´s disease.

The compounds of this invention may be administered in the form of free bases or their salts with non-toxic acids. Some typical examples of these salts are the hydrobromide, hydrochloride, phosphate, sulphate, citrate, and tartrate.

Pharmaceutical preparations

In clinical practice the compounds of the present invention will normally be administered orally, rectally or by injection, in the form of pharmaceutical preparations comprising the active ingredient either as a free base or as a pharmaceutically acceptable non-toxic, acid addition salt, e.g. the hydrochloride, hydrobromide, lactate, acetate, sulphate, sulphamate and the like in association with a pharmaceutically acceptable carrier. Accordingly, terms relating to the novel compounds of this invention whether generically or specifically are intended to inlcude both the free amine base and the acid addition salts of the free base, unless the context in which such terms are used, e.g. in the specific examples would be inconsistent with the broad concept. The carrier may be a solid, semisolid or liquid diluent or capsule. These pharmaceutical preparations constitute a further aspect of this invention. Usually the active substance will constitute between 0.1 and 99 % by weight of the preparation, more specifically between 0.5 and 20 % by weight for preparation intended for injection and between 2 and 50 % by weight for preparations suitable for oral administration.

To produce pharmaceutical preparations containing a compound of the invention in the form of dosage units for oral application, the selected compound may be mixed with a solid pulverulent carrier, e.g. lactose, saccharose, sorbitol, mannitol, starches such as potato starch, corn starch or amylopectin, cellulose derivatives, or gelatine, and a lubricant such as magnesium stearate, calcium stearate, polyethylene glycol waxes, and the like, and then compressed to form tablets. If coated tablets are required, the cores, prepared as described above, may be coated with a concentrated sugar solution which may contain, e.g. gum arabic, gelatine, talcum, titanium dioxide, and the like. Alternatively, the tablet can be coated with a lacquer dissolved in a readily volatile organic solvent or mixture of organic solvents. Dyestuffs may be added to these coatings in order to readily distinguish between tablets containing different active substances of different amounts of the active compound.

For the preparation of soft gelatine capsules (pearl-shaped closed capsules) consisting of gelatine and for example, glycerol or similar closed capsules, the active substance may be admixed with a vegetable oil. Hard gelatine capsules may contain granulates of the active substance in combination with solid, pulverulent carriers such as lactose, saccharose, sorbitol, mannitol, starches (e.g. potato starch, corn starch or amylopectin), cellulose derivatives or gelatine.

Dosage units for rectal application can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions for example, solutions containing from about 0.2 % to about 20 % by weight of the active substance herein described, the balance being sugar and a mixture of

ethanol, water, glycerol, and propyleneglycol. Optionally such liquid preparations may contain colouring agents, flavouring agents, saccharine and carboxymethylcellulose as a thickening agent.

Solutions for parenteral applications by injection can be prepared in an aqueous solution of a water-soluble pharmaceutically acceptable salt of the active substance preferably in a concentration of from about 0.5 % to about 10 % by weight. These solutions may also contain stabilizing agents and/or buffering agents and may conveniently be provided in various dosage unit ampoules.

Suitable peroral doses of the compounds of the invention are 2-20 mg, preferably 5-15 mg given 1 to 3 times a day, preferably 2 times a day.

Best Mode of Carrying Out the Invention

The preferred compound of the invention is the compound of the formula

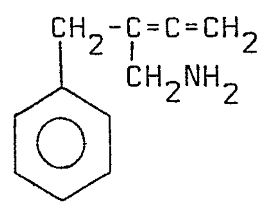

in the form of its acid-addition salt with hydrochloric acid.

Methods of preparation

Reacting an acetylenic aminoether of the formula

$$R\text{-}O\text{-}CH_2\text{-}C\equiv C\text{-}CH_2\text{-}NH_2$$

wherein R is lower alkyl (preferably methyl) or 1-alkoxyalkyl,

with a Grignard, or lithium reagent, of the formula

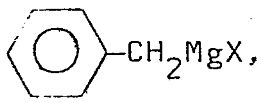 or 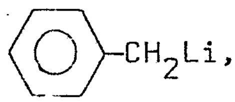 in the presence of a Cu(I)-salt as a catalyst.

The reaction is conducted in a suitable solvent e.g. ethyl ether. Further information about this type of reaction can be found in Tetrahedron Letters No. 15, pp 1319-1322, 1978, and in the following working examples.

Intermediates

For the preparation of the compounds according to the invention it has been found that the compound of the formula

$$R-O-CH_2-C\equiv C-CH_2-NH_2$$

wherein R is a lower alkyl group, preferably methyl, is a valuable starting material.

The preparation of intermediates of this type is illustrated in Example 1 hereinbelow.

Working examples

Preparation of starting materials

Example 1. 4-Methoxy-2-butynamine

Methylpropargylether (25 g), paraformaldehyde (14 g), dibutylamine (56 g), copper (I) chloride (0.5 g), and dioxane (200 ml) were mixed and then heated to reflux during three hours. After cooling, the mixture was poured into water and the obtained product (N,N-dibutyl-4-methoxy-2-butynamine) was

extracted with ether. After drying over $K_2CO_3$, the product was distilled. Yield: 70 g, b.p. 80-82$^oC$/0.5 mm Hg.

N,N-Dibutyl-4-methoxy-2-butynamine (70 g) was added dropwise to 35 g cyanogen bromide in 400 ml ethyl ether. After stirring for 15 hours at room temperature, the solution was washed two times with aqueous HCl (10 %). After drying over $MgSO_4$ and distillation, the product 4-methoxy-2-butynylbromide was obtained. Yield: 38.9 g, b.p. 67-72$^oC$/10 mm Hg.

4-Methoxy-2-butynylbromide (13 g) was added dropwise to 100 ml liquid ammonia. The mixture was left for two hours in a closed vessel. After evaporation of the ammonia, water was added and the mixture was then extracted several times with ethyl ether. The title product, 4-methoxy-2-butynamine, was then obtained by distillation. Yield: 4.5 g, b.p. 70-72$^oC$/12 mm Hg.

NMR ($CDCl_3$)$\delta$ = 4.05 (triplet, 2H), 3.45 (triplet, 2H), 3.35 (singlet, 3H), 1.45 (singlet, 2H).

Preparation of end compounds

Example 2. 2-Benzyl-2,3-butadienamine

4-Methoxy-2-butynamine (4.5 g) was added dropwise at -30$^oC$ to benzylmagnesiumchloride (obtained from 29 g benzylchloride and 5.5 g magnesium) dissolved in ethyl ether. After stirring for 15 min. in nitrogen atmosphere, copper (I)-bromide (3.3 g) was added at -30$^oC$. The mixture was allowed to attain room temperature. After stirring for 15 hours, the mixture was hydrolyzed with ice and conc. ammonia. The water phase was then repeatedly extracted with ethyl ether. The amine was re-extracted with diluted HCl (aq). After making the mixture alkaline with potassium carbonate, the mixture was extracted with methylene chloride. The product was distilled. Yield: 1.8 g, b.p. 90-96$^oC$/1 mm Hg.

The amine was treated with HCl in ethyl ether, whereby the hydrochloride salt was obtained. The salt was recrystallized from a mixture of ethanol and ethyl ether. Yield: 1.6 g, m.p. 143-144°C.

Analysis: Calculated for $C_{11}H_{14}NCl$: C 67.51; H 6.69; N 7.2.
Found: C 67.45; N 7.3.

Pharmacological test

It is not possible by experimental means to induce depressions in laboratory animals. In order to evaluate a possible antidepressive effect of new substances biochemical-pharmacological test methods must be resorted to. One such method, which seems to give good indication of the potential antidepressive effects of the test substances, is a test for monoamine oxidase enzyme inhibiting activity. The deamination of [$^3$H]phenethylamine, [$^3$H]tyramine, and [$^{14}$C]-5-hydroxytryptamine by brain slices was determined according to the following method.

Slices (100 mg) of the central part of the mouse brain including hypothalamus, thalamus, and midbrain were incubated for 5 min with $1 \times 10^{-7}$ M of the labeled substrate in 2.0 ml of Krebs-Henseleit's buffer, pH 7.4, containing 11.2 µmol of glucose. The slices were then homogenized in an all-glass homogenizer with 1.0 ml of 1 N HCl containing carrier amounts (1 µg/ml) of the nonlabeled amine and its deaminated acid product. An aliquot of 1.1 ml of a brain homogenate in 10 vol of 1 N HCl containing the substrate and product carriers was added to 0.5 ml of the incubation medium. The brain homogenate was added in order to facilitate the extraction of tyramine and 5-HT. The acid and neutral products were extracted into 6.0 ml of ethyl acetate by vigorous shaking for 10 s. After centrifugation, 4.0 ml of the organic phase was taken for determination of radioactivity. The remaining ethyl acetate was removed; the water phase was made alkaline with solid sodium carbonate and 1.0 ml of 0.5 M borate buffer, pH 10. The amine was then extracted into 6.0 ml

of ethyl acetate and 4.0 ml of the organic phase was taken for the assay. The deamination of [$^3$H]tyramine and [$^{14}$C]-5-HT was determined simultaneously by utilizing the double-labeling technique. (S.B. Ross, A.L. Renyi, and S.-O. Ögren, Eur. J. Pharmacol., 17, 107 (1972)).

Inhibition of the MAO activity in the brain slices was determined 1 h after the intraperitoneal injection of the test compound. The inhibition was expressed in percent and calculated on the total formation of acid and neutral products formed in the brain slices of animals treated with the test compounds or with saline.

The results are depicted in the following tables. The first series of tables relate to tests with the compound ACS 104, i.e. the compound 2-benzyl-2,3-butadienamine hydrochloride according to the invention.

Deamination of phenethylamine

| Dose mg/kg | % inhib. | $ED_{50}$ µmol/kg |
|---|---|---|
| 1 h after adm. | | |
| 29 | 92±1 | 16 |
| 4 | 67±2 | |
| 1 | 19±3 | |
| 24 h after adm. | | |
| 29 | 39±2 | |

Deamination of tyramine

| Dose mg/kg | % inhib. | $ED_{50}$ µmol/kg |
|---|---|---|
| 1 h after adm. | | |
| 29 | 63±1 | <150 |

Deamination of 5-hydroxytryptamine

| Dose mg/kg | % inhib. | $ED_{50}$ µmol/kg |
|---|---|---|
| 1 h after adm. | | |
| 29 | 48±2 | ~150 |

The following series of tables relate to tests with the compound ACS 64, i.e. a prior art compound, N-2,3-butadienyl--N-methylbenzylamine hydrochloride, disclosed in US Patent No. 3,397,236.

Deamination of phenethylamine

| Dose mg/kg | % inhib. | $ED_{50}$ µmol/kg |
|---|---|---|
| 1 h after adm. | | |
| 32 | 86±3 | 50 |
| 4 | 31±7 | |
| 24 h after adm. | | |
| 32 | 70±1 | |

Deamination of tyramine

| Dose mg/kg | % inhib. | $ED_{50}$ µmol/kg |
|---|---|---|
| 1 h after adm. | | |
| 32 | 20±3 | >150 |

Deamination of 5-hydroxytryptamine

| Dose mg/kg | % inhib. | $ED_{50}$ µmol/kg |
|---|---|---|
| 1 h after adm. | | |
| 32 | 16±2 | >150 |

A comparison of the results for the compounds ACS 104 and ACS 64 as regards $ED_{50}$-values (i.e. effective dose for 50 % inhibition) shows that the tested compound of the invention is about three times as potent as the prior art compound with regard to inhibition of the MAO activity against phenethylamine. Both the tested compounds were only weak inhibitors of the MAO activity against tyramine and 5-hydroxytryptamine, the compound of invention, however, being somewhat more potent than the prior art compound.

Selective MAO inhibitors of the B-type, to which the invented compounds belong (inhibiting the deamination of phenethylamine but very weakly that of 5-hydroxytryptamine) may in combination with l-dopa also be clinically useful in the treatment of Parkinson´s disease (Birkmayer et. al. J. Neural. Transm. 36, 303-326, 1975).

Claims

1. A compound of the formula

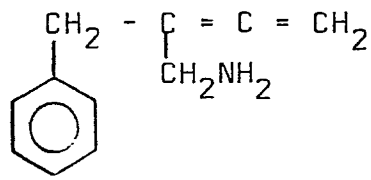

and pharmaceutically acceptable acid-addition salts thereof.

2. A compound according to claim 1 in the form of its acid--addition salt with hydrochloric acid.

3. A compound according to claim 1 in association with a pharmaceutically acceptable carrier.

4. A pharmaceutical preparation which comprises as active ingredient a therapeutically effective amount of a compound of the formula

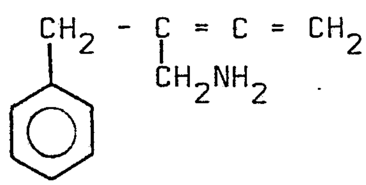

or a pharmaceutically acceptable acid-addition salt thereof, in association with a pharmaceutically acceptable carrier.

5. A pharmaceutical preparation according to claim 4 in dosage unit form.

6. The use of a compound of the formula

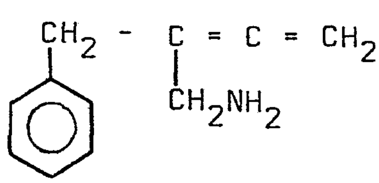

or a pharmaceutically acceptable acid-addition salt thereof, as active ingredient for the preparation of pharmaceutical preparations having antidepressive activity.

7. A process for the preparation of a compound of the formula

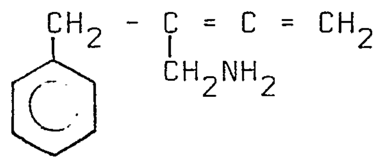

or a pharmaceutically acceptable acid-addition salt thereof, characterized in reacting an acetylenic aminoether of the formula

$$R-O-CH_2-C\equiv C-CH_2-NH_2$$

wherein R is lower alkyl or 1-alkoxyalkyl, with a Grignard, or lithium reagent, of the formula

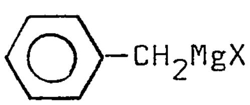, or 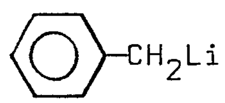, in the presence of a Cu(I)-salt as a catalyst.

European Patent Office

# EUROPEAN SEARCH REPORT

0011608
Application number

EP 79850076.5

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | GB - A - 1 153 578 (CHINOIN)<br>+ Claim 1 +<br>--<br>DE - A - 1 668 830 (VEB-FAHLBERG)<br>+ Claim 1 +<br>--<br>US - A - 3 210 424 (DOMINIC D. MICUCCI)<br>+ Column 1 +<br>--<br>GB - A - 967 711 (GEBR. GIULINI)<br>+ Page 1 and 2 +<br>---- | 1<br><br><br>1<br><br><br>1 - 6<br><br><br>1 - 6 | C 07 C 87/29<br>A 61 K 31/135<br><br>TECHNICAL FIELDS SEARCHED (Int.Cl.3)<br>C 07 C 87/00<br>A 61 K 31/00<br><br>CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| VIENNA | 17-12-1979 | REIF |

EPO Form 1503.1 06.78